(19) Europäisches Patentamt
European Patent Office
Office européen des brevets

(11) Veröffentlichungsnummer: **0 025 934**
**B1**

(12) # EUROPÄISCHE PATENTSCHRIFT

(45) Veröffentlichungstag der Patentschrift: **29.12.82**

(51) Int. Cl.³: **C 07 C 87/56,** C 07 C 85/20

(21) Anmeldenummer: **80105378.6**

(22) Anmeldetag: **09.09.80**

(54) **Verfahren zur Herstellung von p-substituierten, aromatischen Aminen.**

(30) Priorität: **22.09.79 DE 2938376**

(43) Veröffentlichungstag der Anmeldung: **01.04.81 Patentblatt 81/13**

(45) Bekanntmachung des Hinweises auf die Patenterteilung: **29.12.82 Patentblatt 82/52**

(84) Benannte Vertragsstaaten: **BE CH DE FR GB LI NL**

(56) Entgegenhaltungen:
**CH-A-193 616**
**CH-A-201 513**
**US-A-2 092 973**
**US-A-3 761 520**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Nestler, Gerhard, Dr., Rubensstrasse 25, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von p-substituierten, aromatischen Aminen

Die Erfindung betrifft ein neues Verfahren zur Herstellung von p-substituierten, aromatischen Aminen durch Umsetzung von p-substituierten, aromatischen Carbaminsäureestern mit aromatischen Aminen in Gegenwart aliphatischer oder cycloaliphatischer Alkohole.

Es ist aus US-PS 2 159 370 bekannt, durch Ammonolyse von alkylierten Halogenbenzolen bei 150 bis 250°C kernalkylierte Aniline herzustellen. Man erhält ein Isomerengemisch der entsprechenden alkylsubstituierten Aniline — vor allem m-Alkylanilin.

Die GB-PS 846 226 beschreibt die Alkylierung von Anilinen mit Isobutylen bei 150 bis 300°C in Gegenwart von aktivierten Bleicherden vom Montmorillonit-Typ. Je nach der Reaktionstemperatur entstehen Isomerengemische oder vorwiegend p-alkylierte Aniline. Die DE-AS 1 051 271 beschreibt dagegen die Bildung von ortho-alkylierten Anilinen und eine geringe N-Alkylierung durch Umsetzung von aromatischen Aminen mit Olefinen bei 150 bis 350°C in Gegenwart von Friedel-Crafts-Katalysatoren, Schwefelsäure, Phosphorsäure oder Bleicherden (Montmorillonite). Die US-PS 2 115 884 zeigt die Herstellung von N-alkylierten, aromatischen Aminen durch Umsetzung der entsprechenden Amine mit Olefinen in Gegenwart von säureaktivierten Bleicherden, Tonerden, Kaolinen und anderen Silikaten. Die US-PS 3 230 257 lehrt Kern- und N-Alkylierung der Halogenwasserstoffsalze von aromatischen Aminen durch Umsetzung mit Olefinen bei 200 bis 400°C. DE-AS 1 048 277 und DE-AS 1 056 138 beschreiben ebenfalls die Bildung von ortho-kernalkylierten Anilinen bei der Einwirkung von Olefinen auf Aniline in Gegenwart von Friedel-Crafts-Katalysatoren oder Bleicherden bei gleichzeitiger Anwesenheit von Aluminium oder von Aluminiumverbindungen aromatischer Amine bzw. von Alkali- oder Erdalkalimetallen oder deren Aniliden bei 250 bis 350°C und 100 bis 200 atü.

Der Nachteil der vorgenannten Verfahren besteht darin, daß bei relativ hohen Temperaturen gearbeitet werden muß. Viele Olefine geben bei diesen Bedingungen zu Nebenreaktionen, beispielsweise Oligomerisierung oder Umlagerungen, Anlaß. Eine hohe Reaktionstemperatur verlangt außerdem bei Verwendung leichtflüchtiger Stoffe hohe Drücke und somit technisch aufwendige Apparaturen. Außerdem sind die Umsetzungen nicht selektiv. Neben ortho-, meta- und para-Isomeren treten N-alkylierte Amine, zum Teil in beträchtlicher Menge, auf.

Die US-PS 3 123 644 beschreibt die Herstellung von para-alkylierten aromatischen Aminen durch Umsetzung von polyalkylierten, aromatischen Aminen mit in para-Stellung unsubstituierten, aromatischen Aminen bei 150 bis 350°C unter Druck in Gegenwart von Aluminiumsilikaten. Nachteilig ist die aufwendige Herstellung der polyalkylierten, aromatischen Amine. Die Umwandlung beträgt lediglich 61 Prozent, bezogen auf das polyalkylierte, aromatische Amin.

In der US-PS 2 507 755 wird die Herstellung von p-Alkylanilinen durch Umsetzung von Anilin mit Alkylphenolen bei 100 bis 250°C und 30 bis 140 bar in Gegenwart von $ZnCl_2$ allein oder in Kombination mit Anilinhydrohalogeniden beschrieben. Nachteilig ist die aufwendige, umweltbelastende Aufarbeitung mit Salzsäure und Basen. Die Umwandlung beträgt lediglich 11 Prozent, bezogen auf Anilin, die Selektivität 63 Prozent.

Es wurde nun gefunden, daß man p-substituierte, aromatische Amine der Formel

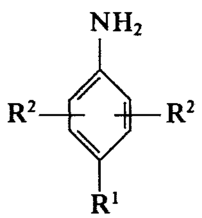

(I)

worin die einzelnen Reste $R^1$ und $R^2$ gleich oder verschieden sein können und jeweils einen araliphatischen oder aliphatischen Rest bedeuten, $R^1$ auch einen monocyclischen oder bicyclischen cycloaliphatischen Rest bezeichnet, $R^2$ auch jeweils für einen aliphatisch-aromatischen oder aromatischen Rest, ein Wasserstoffatom oder ein Halogenatom steht, vorteilhaft erhält, wenn man p-substituierte, aromatische Carbaminsäureester der Formel

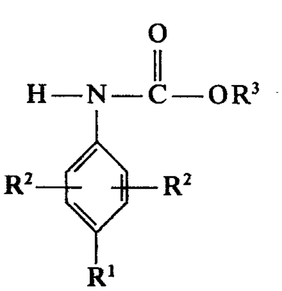

(II)

worin $R^1$ und $R^2$ die vorgenannte Bedeutung besitzen und $R^3$ einen aliphatischen Rest bedeutet, mit aromatischen Aminen der Formel

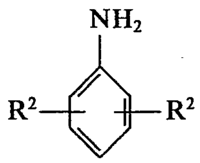

(III)

worin $R^2$ die vorgenannte Bedeutung besitzt, in Gegenwart von aliphatischen Alkoholen der Formel

$R^4$—OH (IV)

worin $R^4$ einen aliphatischen oder cycloaliphatischen Rest bezeichnet, umsetzt.

Die Umsetzung kann für den Fall der Verwendung von Anilin und N-(p-tert.-Butylphenylcarbaminsäureäthylester durch die folgenden Formeln wiedergegeben werden:

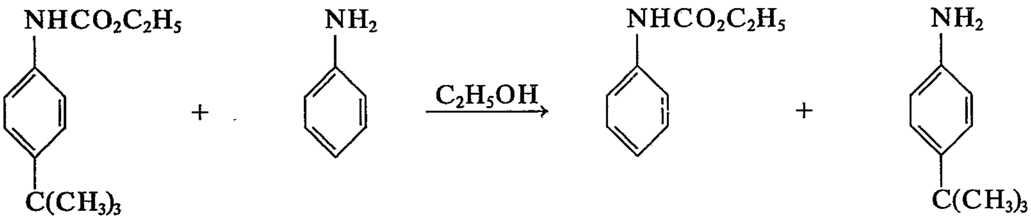


Im Vergleich zu den bekannten Verfahren liefert das Verfahren nach der Erfindung überraschend auf einfacherem und wirtschaftlicherem Weg eine große Zahl von p-substituierten, aromatischen Aminen in besserer Ausbeute, Raum-Zeit-Ausbeute und Reinheit. Im Gegensatz zu den bekannten Verfahren zur Spaltung von Carbaminsäureestern geht bei diesem Verfahren weder die Alkoxycarbonylgruppe verloren, noch werden Säuren oder größere Mengen Base nötig, die bei der Aufarbeitung zu einer Abwasserbelastung führen. So kann man den als Nebenprodukt anfallenden, in p-Stellung unsubstituierten Carbaminsäureester erneut mit Olefinen zu den Ausgangsstoffen II umsetzen, die so erneut für die erfindungsgemäße Reaktion verwendet werden können. Alle diese vorteilhaften Ergebnisse sind im Hinblick auf den Stand der Technik überraschend. Denn es ist aus Houben-Weyl, Methoden der Organischen Chemie, Band 11/1, Seiten 948 und 949, bekannt, daß Carbaminsäureester sowohl in saurem Medium, vorzugsweise mit starker Salzsäure oder Schwefelsäure, als auch unter alkalischen Bedingungen, vorzugsweise in alkoholischer Lösung mit Alkalihydroxyden, bei erhöhter Temperatur gespalten werden können. Außerdem können die den Carbaminsäureestern zugrundeliegenden Amine durch Destillation mit gelöschtem Kalk oder durch Erhitzen auf 200 bis 250°C mit Wasser unter Druck freigesetzt werden (Houben-Weyl, loc. cit., Seite 949). Da bekannt ist, daß die Umsetzung von Carbaminsäureestern mit Aminen bei Temperaturen über 140°C zur Herstellung von Harnstoffen verwendet wird (Houben-Weyl, loc. cit., Band 8, Seite 161), war es überraschend, daß bei der Umsetzung von Carbaminsäureestern mit Aminen in Gegenwart von Alkoholen Harnstoffe nicht in wesentlichen Mengen gebildet werden.

Das Anilin III kann mit dem p-substituierten, aromatischen Carbaminsäureester II in stöchiometrischer Menge, im Überschuß oder Unterschuß, vorzugsweise in einem Verhältnis von 4 bis 20, insbesondere 8 bis 16 Mol Ausgangsstoff III je Mol Carbaminsäureester II umgesetzt werden. Der Alkohol IV wird zweckmäßig in einer Menge von 1 bis 20, vorteilhaft von 1 bis 15, insbesondere 3 bis 10 Mol Alkohol IV je Mol Carbaminsäureester II verwendet. Bevorzugte aromatische Carbaminsäureester II, Aniline III, Alkohole IV und dementsprechend bevorzugte aromatische Amine I sind solche, in deren Formeln die einzelnen Reste $R^1$, $R^2$, $R^3$ und $R^4$ gleich oder verschieden sein können und jeweils einen Alkylrest mit 1 bis 12, insbesondere 1 bis 6 Kohlenstoffatomen bedeuten, $R^1$ auch einen Cycloalkylrest mit 5 bis 8 Kohlenstoffatomen, einen Norbornylrest oder einen Aralkylrest mit 7 bis 12 Kohlenstoffatomen bezeichnet und insbesondere auch einen Alkylrest mit 3 bis 6 Kohlenstoffatomen bedeutet, $R^2$ auch für ein Wasserstoffatom, ein Bromatom oder ein Chloratom einen Alkylarylrest oder Aralkylrest mit 7 bis 12 Kohlenstoffatomen oder einen Phenylrest steht, $R^4$ auch einen Cycloalkylrest mit 5 bis 8 Kohlenstoffatomen bezeichnet. Die vorgenannten Reste können noch durch unter den Reaktionsbedingungen inerte Gruppen, z. B. Alkylgruppen oder Alkoxygruppen mit 1 bis 4 Kohlenstoffatomen, den Phenylrest substituierende Chlor- oder Bromatome, substituiert sein. In einer bevorzugten Ausführungsform verwendet man solche Ausgangsstoffe II, III und IV, in deren Formeln die einzelnen Reste $R^2$ in den Ausgangsstoffen III dieselbe Bedeutung wie die entsprechenden Reste $R^2$ der Ausgangsstoffe II besitzen und $R^4$ die Bedeutung von $R^3$ besitzt, d. h., das Amin III und der Alkohol IV liegen dem Carbaminsäureester II zugrunde.

Beispielsweise sind folgende aromatische Amine III geeignet: Anilin; in 2-, 3-, 5-, 6-Stellung einfach

oder in 2,3-, 2,5-, 2,6-Stellung zweifach gleich oder unterschiedlich durch ein Chloratom, ein Bromatom, eine Methyl-, Äthyl-, Propyl-, Isopropyl-, Butyl-, Isobutyl-gruppe substituierte Aniline; bevorzugt sind Anilin, o-Toluidin, m-Toluidin, o-Chloranilin, m-Chloranilin, o-Äthylanilin, o-Propylanilin, 2-Chlor-6-methylanilin, 5-Chlor-2-methylanilin, 2,5-Dimethylanilin.

Für die Reaktion geeignete aromatische Carbaminsäureester II sind beispielsweise: Der in p-Stellung zur Carbamidogruppe am Phenylkern durch die tert.-Butylgruppe substituierte Carbamidsäuremethylester der im vorstehenden Absatz beispielsweise genannten Aniline III; homologe Äthyl-, Propyl-, Isopropyl-, Butyl-, sek.-Butyl-, Isobutyl-, Cyclohexyl-, Cyclopentylester vorgenannter Carbamidsäureester; entsprechende in p-Stellung durch die Äthyl-, Methyl-, Propyl-, Isopropyl-, Butyl-, sek.-Butyl-, Pentyl-, Isopentyl-, Isoheptyl-, Benzyl-, $\alpha$-Phenyläthyl-, $\beta$-Phenyläthyl-, Cumyl-, Norbornyl-, Cyclohexyl-, Cyclopentyl-gruppe substituierte Carbamidsäureester; bevorzugt sind p-tert.-Butylphenyl-, 4-tert.-Butyl-2-methylphenyl-, 4-tert.-Butyl-2-chlorphenyl-, 4-tert.-Butyl-5-chlor-2-methylphenyl-, 4-tert.-Amylphenyl-, p-Cyclohexylphenyl-, p-Cyclopentylphenyl-, 4-Cyclopentyl-2-methylphenyl-, 4-($\alpha$-Phenyläthyl)-phenyl-, 2-Chlor-4-($\alpha$-phenyläthyl)-phenyl-, 4-Cumylphenyl-, 4-Benzylphenyl-4-Norbornylphenyl-, 2-Chlor-4-norbornylphenylcarbaminsäuremethyl-ester und die entsprechenden Ätyhl-, Propyl-, Butyl-ester.

Für die Umsetzung geeignete Alkohole IV sind beispielsweise: Die den im vorstehenden Absatz beispielsweise genannten Carbaminsäureestern II zugrundeliegenden Alkohole, insbesondere Methanol, Äthanol, Propanol, Butanol, Isopropanol.

Die Umsetzung wird im allgemeinen bei einer Temperatur von 100 bis 260° C, vorzugsweise von 130 bis 250° C, insbesondere 150 bis 220° C, mit Unterdruck, Überdruck oder drucklos, kontinuierlich oder diskontinuierlich durchgeführt. Zweckmäßig verwendet man keine zusätzlichen Lösungsmittel; gegegebenenfalls kommen aber auch, z. B. zur Erniedrigung der Viskosität des Reaktionsgemisches, unter den Reaktionsbedingungen inerte Lösungsmittel in Betracht. Als Lösungsmittel kommen z. B. in Frage: aliphatische oder cycloaliphatische Kohlenwasserstoffe, z. B. Heptan, Nonan, Benzinfraktionen innerhalb eines Siedepunktintervalls von 70 bis 190°C, Cyclohexan, Methylcyclohexan, Ligroin, Halogenkohlenwasserstoffe, insbesondere Chlorkohlenwasserstoffe, z. B. Tetrachloräthylen, Tetrachlorkohlenstoff, Chlorbenzol, aromatische Kohlenwasserstoffe, z. B. Benzol, Toluol, Xylole; Tetrahydrofuran, Dioxan; und entsprechende Gemische. Zweckmäßig verwendet man das Lösungsmittel in einer Menge von 10 bis 1000 Gewichtsprozent, vorzugsweise von 50 bis 200 Gewichtsprozent, bezogen auf Ausgangsstoff II.

In einer vorteilhaften Ausführungsform wird die Umsetzung in Gegenwart einer basischen Verbindung vorteilhaft in einer Menge von 0,001 bis 0,2, vorzugsweise von 0,005 bis 0,1 Äquivalenten basischen Verbindung, bezogen auf ein Mol Ausgangsstoff II, durchgeführt. Bevorzugte basische Verbindungen sind Erdalkaliverbindungen, Alkaliverbindungen und tertiäre Amine sowie entsprechende Gemische. Als tertiäre Amine kommen aliphatische, cycloaliphatische, araliphatische, aromatische und heterocyclische Amine in Betracht. Es kommen z. B. als basische Verbindungen in Frage: Kaliumhydroxid, Natriumhydroxid, Kaliumcarbonat, Natriumcarbonat, Lithiumhydroxid, Lithiumcarbonat, Calciumhydroxid, Calciumoxid, Bariumoxid, Magnesiumhydroxid, Magnesiumoxid, Bariumhydroxid, Natriummethylat, Natriumäthylat, Natriumpropylat, Natriumisopropylat, Natriumbutylat, Kaliummethylat, Kaliumäthylat; Triäthylamin, Tripropylamin, Triisopropylamin, Tributylamin, N,N-Dimethylanilin, 1,4-Diazabicyclo-(2,2,2)-octan, 1,5-Diazabicyclo-(4,3,0)-non-5-en, N-Methylpiperidin, N-Äthylpiperidin, Pyridin. Bevorzugt sind Natriummethylat, Natriumäthylat, Natriumhydroxid und Kaliumhydroxid.

Die Reaktion kann wie folgt durchgeführt werden: Ein Gemisch von Ausgangsstoff II und III, Alkohol IV und gegebenenfalls Katalysator und/oder Lösungsmittel wird während 1 bis 10 Stunden bei der Reaktionstemperatur gehalten. Aus dem Reaktionsgemisch wird der Endstoff I in üblicher Weise, z. B. durch fraktionierte Destillation, abgetrennt.

Die nach dem Verfahren der Erfindung herstellbaren aromatischen Amine I sind Antiseptika und wertvolle Ausgangsstoffe für die Herstellung von Farbstoffen, Harzen, Lacken, Kunststoffen, Schädlingsbekämpfungsmittel und Pharmazeutika. Sie und ihre Folgeprodukte können als Antioxidantien bei der Herstellung von synthetischem Gummi und Ausgangsstoffe zur Herstellung von Riechstoffen und Wirkstoffen verwendet werden. Bezüglich der Verwendung wird auf US-PS 3 123 644, GB-PS 846 226, JP-PS 4 849 925, BE-PS 757 983, die vorgenannten Veröffentlichungen und Ullmanns Encyklopädie der technischen Chemie (3. Auflage), Band 5, Seiten 300 bis 357, verwiesen.

Die in den folgenden Beispielen aufgeführten Teile bedeuten Gewichtsteile. Sie verhalten sich zu den Volumenteilen wie Kilogramm zu Liter.

## Beispiel 1

In einem Autoklaven wird ein Gemisch aus 110 Teilen N-(4-tert.-Butylphenyl)-carbaminsäureäthylester, 370 Teilen Anilin und 700 Teilen Äthanol auf 180°C erhitzt und 6 Stunden bei dieser Temperatur gehalten. Anschließend wird das Gemisch im Vakuum fraktionierend destilliert. Man erhält 60 Teile (80% der Theorie) 4-tert.-Butylanilin vom Kp 93 bis 94° C (4 mbar).

Beispiel 2

In einem Autoklaven wird ein Gemisch aus 110 Teilen N-(4-tert.-Pentylphenyl)-carbaminsäuremethylester, 400 Teilen Anilin, 400 Teilen Methanol und 0,2 Teilen Natriummethylat auf 200°C erhitzt und 4 Stunden bei dieser Temperatur gehalten. Anschließend wird das Gemisch im Vakuum fraktionierend destilliert. Man erhält 67 Teile (83% der Theorie) 4-tert.-Pentylanilin vom Kp 140 bis 142°C (16 mbar).

**Patentanspruch:**

Verfahren zur Herstellung von p-substituierten, aromatischen Aminen der Formel

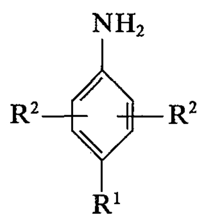

(I)

worin die einzelnen Reste $R^1$ und $R^2$ gleich oder verschieden sein können und jeweils einen araliphatischen oder aliphatischen Rest bedeuten, $R^1$ auch einen monocyclischen oder bicyclischen cycloaliphatischen Rest bezeichnet, $R^2$ auch jeweils für einen aliphatisch-aromatischen oder aromatischen Rest, ein Wasserstoffatom oder ein Halogenatom steht, dadurch gekennzeichnet, daß man p-substituierte, aromatische Carbaminsäureester der Formel

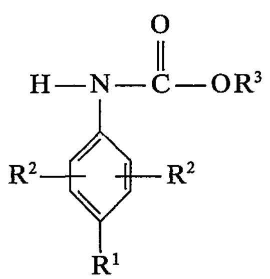

(II)

worin $R^1$ und $R^2$ die vorgenannte Bedeutung besitzen und $R^3$ einen aliphatischen Rest bedeutet, mit aromatischen Aminen der Formel

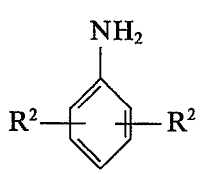

(III)

worin $R^2$ die vorgenannte Bedeutung besitzt, in Gegenwart von aliphatischen Alkoholen der Formel

$R^4$—OH (IV)

worin $R^4$ einen aliphatischen oder cycloaliphatischen Rest bezeichnet, umsetzt.

**Claim**

A process for the preparation of p-substituted, aromatic amines of the formula

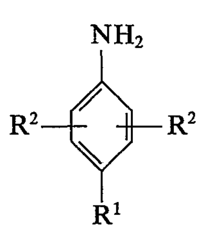

(I)

where the individual radicals $R^1$ and $R^2$ may be identical or different and each is an araliphatic or aliphatic radical, $R^1$ may also be a monocyclic or bicyclic cycloaliphatic radical and each $R^2$ may also be an aliphatic-aromatic or aromatic radical, hydrogen, wherein a p-substituted, aromatic carbamic acid ester of the formula

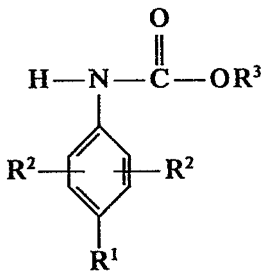 (II)

where $R^1$ and $R^2$ have the above meanings and $R^3$ is an aliphatic radical, is reacted with an aromatic amine of the formula

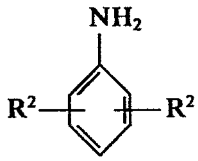 (III)

where $R^2$ has the above meanings, in the presence of an aliphatic alcohol of the formula

$R^4$—OH (IV)

where $R^4$ is an aliphatic or cycloaliphatic radical.

**Revendication**

Procédé pour la préparation d'amines aromatiques p-substituées de formule

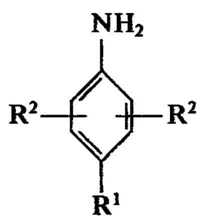 (I)

dans laquelle les différents radicaux $R^1$ et $R^2$ peuvent être semblables ou différents et représentent chacun un radical araliphatique ou aliphatique, $R^1$ pouvant aussi désigner un radical cycloaliphatique monocyclique ou bicyclique, $R^2$ pouvant être également mis pour un radical aliphatique-aromatique ou aromatique, un atome d'hydrogène ou un atome d'halogène, caractérisé en ce qu'on fait réagir des carbamates aromatiques p-substitués de formule

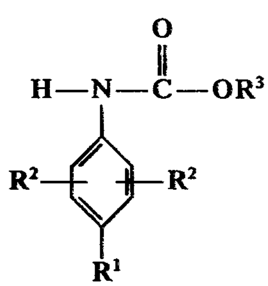 (II)

dans laquelle $R^1$ et $R^2$ ont les significations données ci-dessus et $R^3$ représente un radical aliphatique, avec des amines aromatiques de formule

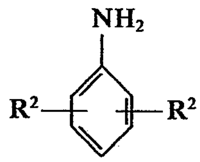

(III)

dans laquelle $R^2$ a la signification donnée ci-dessus, en présence d'alcools aliphatiques de formule

$$R^4—OH \quad (IV)$$

dans laquelle $R^4$ désigne un radical aliphatique ou cycloaliphatique.